# EUROPEAN PATENT SPECIFICATION

(11) **EP 0 996 436 B1**
(45) Date of publication and mention of the grant of the patent: **27.07.2005**
(21) Application number: 98907701.1
(22) Date of filing: 27.02.1998
(51) Int. Cl.: A61K 31/19, A61K 31/47, A61K 9/48, A61K 47/10

(54) **PHARMACEUTICAL FORMULATIONS CONTAINING POORLY SOLUBLE DRUG SUBSTANCES**
PHARMAZEUTISCHE ZUSAMMENSETZUNGEN, DIE SCHWERLÖSLICHE ARZNEISTOFFE ENTHALTEN
PREPARATIONS PHARMACEUTIQUES CONTENANT DES SUBSTANCES MEDICAMENTEUSES FAIBLEMENT SOLUBLES

(30) Priority: 03.03.1997 US 813946; 03.03.1997 US 808761; 03.03.1997 US 810560; 24.02.1998 US 28504
(43) Date of publication of application: 03.05.2000
(73) Proprietor: Sanofi-Aventis, 75013 Paris (FR)
(72) Inventor: ROCCO, William, L., Reading, PA 19606 (US); LAUGHLIN, Sharon, M., Phoenixville, PA 19460 (US); FRANSON, Nancy, M., Collegeville, PA 19426 (US)
(74) Representative: White, Martin Paul
(86) International application number: PCT/US1998/004078
(87) International publication number: WO 1998/038987

(56) References cited:
- WO-A-93/11753
- DD-A- 293 499
- US-A- 4 623 648
- US-A- 4 725 427
- US-A- 5 071 643
- US-A- 5 376 688
- US-A- 5 420 141
- US-A- 5 681 606

## Description

### Background of the Invention

### 1. Field of the Invention

This invention relates to pharmaceutical formulations comprising a poorly soluble drug substance and a pharmaceutically acceptable carrier.

### 2. Reported Developments

Poorly soluble drug substances have been formulated in pharmaceutically acceptable carriers by finely dividing them, by grinding, into nanoparticles having an effective average particle size of less than 400 nm in order to achieve satisfactory bioavailabiity. However, to keep the nanoparticles from coagulation various surface active agents were used. Such agents may not be desirable and they also reduce the overall amount of the drug which otherwise could be contained in an effective dosage formulation.

Solid dispersions have also been used to increase the dissolution rate and bioavailability of drugs that are poorly water soluble. The carriers used have been physiologically inert compounds that are readily water soluble, such as polyethylene glycols. Two techniques which have been used to prepare solid dispersions are the fusion technique and the solvent technique. In the fusion technique, the drug substance is dissolved in a molten carrier and the mixture cooled to form a solid. In the solvent technique, drug substance and carrier are dissolved in a solvent, followed by removal of the solvent by evaporation or freeze drying.

The preparation of solid dispersions featuring good pharmaceutical properties is difficult. Problems which frequently occur during preparation include: degradation of drug substance at the temperature of the molten carrier; reaction of the drug with the molten carrier; and incomplete solidification of the product, e.g., the carrier remaining largely amorphous. Solid dispersions prepared from esters of p-aminobenzoic acid and xylitol are disclosed, for example, by Sirenius et al., *J. Pharm. Sci*., 66, No. 6, June 1979.

The present invention is directed to pharmaceutical formulations containing poorly soluble drug substances and particularly to SR 48692 which has shown considerable promise as an NT-antagonist for the treatment of psychosis. SR48692 and a method for the preparation thereof are described by Boigegrain et al in United States Patent No. 5,420,141 (Example 13). SR48692 has the structural formula: 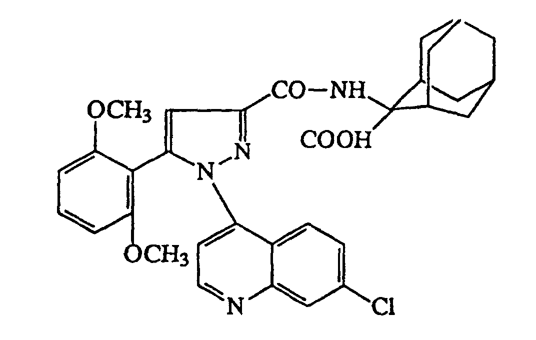 SR48692 has proven to be unusually difficult to formulate into pharmaceutical compositions, due in part to its very low solubility, even in organic solvents.

PCT/US87/02629 discloses a solvent system for enhancing the solubility of an acidic, basic or amphoteric pharmaceutical agent to produce a concentrated solution suitable for soft gelatin capsule filling. The solvent system comprises polyethylene glycol containing 0.2 - 1.0 mole equivalents of an ionizing agent per mole equivalent of pharmaceutical agent and 1-20% water. Attempts to formulate SR48692 in such a solvent system were not successful.

Sheen et al. in *Int. J. Pharm*., 118(2), 221-7, 1995, disclose a solid dispersion of a poorly water-soluble drug, RP69698 prepared by a melting method with water-soluble carriers in which RP69698 is highly soluble. When incorporated into a solid dispersion, the formulation contains PEG 3350, Transcutol and Labrasol.

### SUMMARY OF THE INVENTION

We have now discovered that poorly soluble drug substances may be incorporated into pharmaceutically acceptable carriers containing certain solvents and employing processes disclosed herein.

The invention can be practiced with a wide variety of drug substances. The drug substance preferably is present in an essentially pure form. The drug substance is poorly soluble and must be soluble in at least one liquid medium. By "poorly soluble" it is meant that the drug substance has a solubility in an aqueous medium of less than about 10 mg/ml, and preferably of less than about 1 mg/ml.

Suitable drug substance can be selected from a variety of known classes of drugs including, for example, analgesics, anti-inflammatory agents, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytic sedatives (hypnotics and neuroleptics), astringents, beta-adrenoceptor blocking agents, blood products and substitutes, cardiac inotropic agents, contrast media, corticosteroids, cough suppressants (expectorants and mucolytics), diagnostic agents, diagnostic imaging agents, diuretics, dopaminergics (antiparkinsonian agents), haemostatics, immunological agents, lipid regulating agents, muscle relaxants, parasympathomimetics, parathyroid calcitonin and biphosphonates, prostaglandins, radio-pharmaceuticals, sex hormones (including steroids), anti-allergic agents, stimulants and anoretics, sympathomimetics, thyroid agents, vasodilators and xanthines. Preferred drug substances include those intended for oral administration. A description of these classes of drugs and a listing of species within each class can be found in Martindale, the extra Pharmacopoeia, Twenty-ninth Edition, the Pharmaceutical Press, London, 1989.

The drug substances are commercially available and/or can be prepared by techniques known in the art.

Preferred drug substances for the purposes of the present invention include Naproxyn and compound SR48692.

Naproxyn is (S)-6-methoxy-α-methyl-2-naphthaleneacetic acid, having the chemical structure 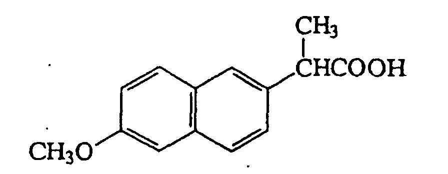 is well known in the prior art, and its preparation is disclosed, for example, in U.S. Patents 3,904,682 and 4,009,197.

SR48692 is disclosed in U.S. Patent No. 5,420,141 in Example 13, having the chemical structure

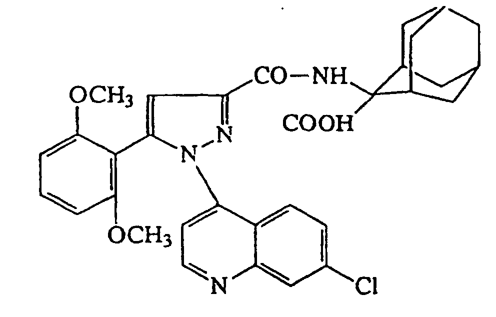

### Solid Dispersions IA and IB

**IA**) We have discovered, as disclosed in copending application Ser. No. 08/813,946, that the combination of Transcutol™ (diethyleneglycol monoethyl ether) and xylitol provides a carrier for solid pharmaceutical dispersions which can reduce impurities and/or degradation products. The dispersion maintains a high degree of crystallinity, acceptable hygroscopicity and rapidly dissolves in water and has good bioavailability.
   In accordance with the invention, there is provided a solid dispersion comprising a poorly soluble drug substance, Transcutol™ and xylitol.
   In another embodiment of the invention, there is provided a method of preparing such dispersion comprising the steps of dissolving a poorly soluble drug substance in Transcutol™ and adding the solution to xylitol.
**IB**) During further research we have discovered that Transcutol™ can be replaced with propylene glycol using a spray granulation process within a fluid bed. The process comprises the steps of:
   a) preparing a drug solution by adding SR48692, or another desired poorly soluble drug, to a system containing propylene glycol and aqueous sodium hydroxide; optionally adding this solution to an aqueous xylitol solution; and
   b) adding the drug solution to a seed powder, such as xylitol, dicalcium phosphate dihydrate, or a combination of lactose monohydrate and microcrystalline cellulose by spraying the drug solution on the seed powder.

### Concentrated Solutions IIA and IIB

**IIA** We have discovered, as disclosed in copending application Ser. No. 08/808,761, that SR48692, or other poorly soluble drug, may be incorporated as the active ingredient in polyethylene glycol (PEG), NaOH, and H₂O wherein the mole equivalent of NaOH per mole equivalent of the active ingredient is at least 1.1.
   The formulations were prepared by adding aqueous NaOH to the polyethylene glycol. The active ingredient SR48692 is added to the PEG-NaOH until a solution is formed. Alternatively, the active ingredient was dispersed in the PEG with mixing. Thereafter, the NaOH solution was added resulting in dissolution of the active ingredient. Thereafter, the solution formulations were encapsulated in a soft gelatin capsule according to techniques known in the art in order to form a pharmaceutical dosage form.
**IIB**) During further research we have discovered that polyethylene glycol may be replaced with propylene glycol wherein the process comprises the steps of:
   a) adding aqueous NaOH to propylene glycol;
   b) adding SR48692, or another poorly soluble organic acid drug, to the aqueous NaOH/propylene glycol to form a solution of the active drug; and
   c) encapsulating of the solution in a soft gelatin capsule.

Alternatively, the active drug can be dispersed in propylene glycol first with mixing followed by the addition of aqueous NaOH to dissolve the active drug.

Yet another embodiment of the invention includes a pharmaceutical formulation comprising:
a poorly soluble drug, propylene glycol, NaOH, and water, wherein the mole equivalent of NaOH per mole equivalent of the poorly soluble drug is at least 1.1.

### Suspensions IIIA and IIIB

**IIIA**) We have now discovered, as disclosed in US Patent No. 5,776,987, that SR48692, or other poorly soluble drugs, may be incorporated as the active ingredient in a solvent system to produce a highly concentrated suspension suitable for hard gelatin capsule filling.
   The suspension comprises:
   0.1 to 40% by weight of SR48692;
   0.1 to 40% by weight of Transcutol™ ;
   0.1 to 99% by weight starch;
   NaOH; and
   H₂O.

   The suspension can be prepared by adding the SR48692 to the solvent Transcutol™, optionally in combination with PEG. The SR48692 is then dissolved by adding aqueous NaOH. The suspension is obtained by adding water which causes a controlled precipitation. Addition of starch to the suspension permits the formulation to be filled into hard gelatin capsules preserving the integrity of the capsule shells.
**IIIB**) During further research we have discovered that Transcutol™ may be replaced with propylene glycol in the suspension to produce a highly concentrated suspension suitable for hard gelatin capsule filling.
   The suspension comprises:
   0.1 to 40% by weight of SR48692;
   0.1 to 40% by weight of propylene glycol;
   0.1 to 99% by weight starch;
   NaOH; and
   H₂O.

   Starch may be replaced with other diluents, such as microcrystalline cellulose and lactose.

### DESCRIPTION OF THE PREFERRED EMBODIMENTS

### Solid Dispersions IA and IB

**IA**) Transcutol™ is also known as diethyleneglycol monoethyl ether. In the dispersions of the invention, Transcutol™ is present in an amount of 0.1 to 30%, preferably 0.5 to 25% and I most preferably, 1 to 20% by weight of the solid dispersion. Transcutol™ is commercially available and/or can be prepared by techniques well known to those skilled in the art.

Xylitol is a pentahydric alcohol derived from xylose having the structure CH₂OH(CHOH)₃CH₂OH and a molecular weight of 152.1. In the dispersions of the invention, xylitol is present in an amount of 40 to 99.9%, preferably 45 to 99.5%, and most preferably 50 to 99% by weight of the solid dispersion. Xylitol is commercially available, and/or can be prepared by techniques well known to those skilled in the art.

In the dispersions of the invention, the drug substance is present in an amount of 0.1 to 30%, preferably 0.5 to 25% and most preferably 1 to 20% by weight of the solid dispersion. In a preferred embodiment the drug substance is SR48692.

### SR48692

Extensive efforts have been made to formulate SR48692 into an acceptable solid dispersion. As described below, attempts to formulate this compound with conventional solid carriers such as high molecular weight polyethylene glycols did not yield satisfactory results.

The dispersions can be prepared by the steps of: dissolving the poorly soluble drug substance in Transcutol™, optionally in the presence of a strong base such as NaOH or KOH, and adding the solution to xylitol to form a crystalline dispersion. The strong base is preferred when the acid form of the drug substance is employed. Up to 1 or more equivalents of base per equivalent of drug substance can be employed. In the final dispersion, the drug substance can be either in a crystalline state or amorphous depending on the drug substance selected and the drug concentration.

The following examples further illustrate the invention.

### Examples 1-4

### Preparation of Solid SR48692 Dispersions and Comparative Examples A-D

The formation of a solution by dissolving SR48692 in a basic solvent system was the initial step. SR48692 was dissolved in PEG 400 or Transcutol™ (diethyleneglycol monoethyl ether available from Gattefosse) with the addition of aqueous sodium or potassium hydroxide at approximately stoichiometric levels with SR48692. Concentrated aqueous sodium hydroxide (35% w/v) or potassium hydroxide (45% w/v) was added to a suspension of SR48692 in the organic solvent Transcutol™ available from Gattefosse; dissolution occurred in less than 15 minutes. Initially, preparations were formed using sodium hydroxide as the base, however, in later formulations potassium hydroxide was used. The basic solution was then added to molten PEG 8000 or xylitol at various levels to produce dispersions at final drug concentration up to approximately 9%. After addition of the SR48692 solution to the carrier, the system was mixed for approximately 2 minutes, then allowed to crystallize overnight at 20°C. Listed below are the combinations which were investigated:

| **Example** | **Solvent** | **Carrier** | **Approx. Drug Conc. (%w/w)** |
|---|---|---|---|
| 1 | Transcutol/NaOH | Xylitol | 3% |
| 2 | Transcutol/NaOH | Xylitol | 5% |
| 3 | Transcutol/KOH | Xylitol | 8% |
| 4 | Transcutol/NaOH | Xylitol | 8% |
| A | Transcutol/NaOH | PEG 8K | 3% |
| B | PEG 400/NaOH | Xylitol | 5% |
| C | PEG 400/NaOH | PEG 8K | 5% |
| D | PEG 400/KOH | Xylitol | 8% |

HPLC results were obtained for measurement of potency as well as to check the impurity levels. The column used was a C18 column with a mobile phase consisting of 0.01M potassium phosphate monobasic (pH=2.5)/acetonitrile (270/730) at a flowrate of 1.0 ml/min. Injection volume was 10 microliters and the detection method was by UV (at 230 nm). The instrument utilized throughout the study was a Hewlett Packard 1050 system. For the assay determination, the samples were first diluted in methanol/ammonium hydroxide (99.8/0.2), then further diluted in methanol to obtain a final SR48692 concentration of 0.04 mg/ml. For the impurity profile, the samples were diluted to a final SR48692 concentration of 1.0 mg/ml by the same procedure. The known impurities were quantitated versus external standards at a concentration of 0.0005 mg/ml. Any unknown impurities were quantified versus an external standard. All impurities greater than 0.01% were added to obtain the total impurity result.

Differential scanning calorimetry (DSC) scans were performed on the Perkin Elmer System-7 at 10°C/min heating rate from 25-125°C under nitrogen purge. Approximately 5 mg samples were encapsulated in aluminum pans. The instrument was calibrated with indium (melting point 156.6°C) and tin (melting point 231.9°C) prior to use.

The dissolution rate of 25 mg SR48692 solid dispersion in hard gelatin capsules were measured in deionized water (900 ml, pH=5.5, unbuffered) at 37°C. A USP dissolution system (Apparatus 2) with a paddle speed of 100 RPM was used. Dissolution rate was monitored by UV (Hewlett Packard 8452A) at 234 nm. The pH was found to be relatively unchanged at the final concentrations (approximately 0.03 mg/ml).

A moisture absorption system was used to investigate the hygroscopicity of a batch of solid dispersion with acceptable impurity levels. The system was run from 5-80% RH in 5% steps at 25°C. The samples were purged at 50°C and about 0% RH prior to analysis.

The xylitol/Transcutol™ formulations containing approximately 8% drug were analysed with a powder x-ray diffraction system in order to determine whether the drug exists in a crystalline or a molecularly dispersed state within the dispersion. Samples were scanned at 2 degrees (2-theta)/minute and compared with pure xylitol. The system was run using Cu k-alpha radiation at 45kv/40ma and a liquid nitrogen cooled solid state germanium detector.

The results obtained for total impurity ievei and the assay for solid dispersions with PEG 8000 or xylitol as the carrier in combination with Transcutol™ or PEG 400 as the solvent are shown in Table 1.

**TABLE 1**

| **Summary of HPLC RESULTS** | | | |
|---|---|---|---|
| **Example** | **% Impurity** | **Assay (%)** | **Nominal Conc. (%)** |
| A | 28.0 | 2.1 | 3 |
| 1 | 0.74 | 2.8 | 3 |
| 2 | 0.40 | 5.2 | 5 |
| B | 1.4 | 5.1 | 5 |
| C | 4.5 | 4.8 | 5 |
| 3 | 0.55 | 9.4 | 8 |
| D | 19.2 | 6.6 | 8 |
| 3 | 0.42 | 8.6 | 8 |
| 3 | 0.36 | 8.8 | 8 |

The total batch size for these samples was about 10 g. At approximately 3% drug concentration, the use of PEG 8000 with Transcutol™ resulted in gross degradation as reflected in the high total impurity level and the lower assay value (Example A). In contrast, the combination of xylitol with Transcutol™ produced a formulation with a significantly lower total impurity level of 0.74% (Example 1).

At approximately 5% drug concentration, the combination of xylitol as the carrier with Transcutol™ as the solvent again produced an acceptable impurity level of 0.40% (Example 2). The total impurity levels for the xylitol/PEG 400 (Example B) and the PEG 8000/PEG 400 (Example C) were 1.4% and 4.5% respectively. Acceptable impurity levels in the final product should approximate impurity levels present in the starting materials. It appeared that the use of PEG 8000 as the carrier or PEG 400 as the solvent resulted in high impurity levels. The carrier/solvent combination of xylitol/ Transcutol™ demonstrated unexpectedly lower impurity levels compared to xylitol/ PEG.

At high drug concentrations (approximately 8-9%), the use of potassium hydroxide versus sodium hydroxide was shown to result in equally low levels of impurity (Example 3). The average impurity level for three 10 gram batches with KOH were 0.44 +/- 0.10%. As was observed previously, the use of PEG 400 as the solvent resulted in unacceptable degradation (19.2% total impurity, Example D).

HPLC data for two batches of approximately 100g made with xylitol as the carrier, Trancutol™ as the solvent and either potassium or sodium hydroxide as the base exhibited low impurity levels; 0.30% (Example 3) and 0.27% (Example 4) total impurities were observed for the KOH and NaOH based systems, respectively.

Differential scanning calorimetry (DSC) scans obtained at 10°C/min for the batches evidenced that the effect of the formulation components on the melting behavior of xylitol is surprisingly low. The melting peak of the KOH based formulation (Example 3) was 89.4°C, a depression of 8.7 degrees. Similarly, the melting peak of the NaOH formulation (Example 4) was 96.3°C, a depression of only 1.8 degrees. X-ray powder diffraction patterns of the formulation indicated a predominantly crystalline xylitol phase.

A screening dissolution test was run in USP Apparatus 2 with water at pH 5.5 (unbuffered) at 37°C comparing the solid dispersion of this invention (Example 2) with unformulated (jet-milled) SR48692. The solid dispersions provide enormous dissolution enhancement with respect to pure drug and low impurity levels (0.4%). The fraction dissolved was well over 90% in 10 minutes; in contrast, the capsules containing SR48692 drug substance (unformulated) did not reach 10% dissolved at the end of the experiment of 60 minutes. Dissolution experiments incorporating the solid dispersion at 25 mg/capsule (Example 2) at pH 7.0 (37°C) with 0.5% sodium lauryl sulfate as the dissolution medium also showed rapid dissolution, with more than 90% dissolved after 10 minutes.

The hygroscopicity of a solid dispersion based formulation (Example 3) was screened by running a sample at 25°C from 5-80% RH in 5% steps. The data showed relatively low (~1%) moisture uptake at 25°C below 60% RH; this level would be acceptable for a solid formulation. Hysteresis data showed the moisture uptake at 80% RH was reversible; as the RH was decreased back down to 5%, the moisture was not retained.

The x-ray powder diffraction patterns of the xylitol/Transcutol™ formulations (Examples 3-4) compared with pure xylitol suggest that the dispersion appears to be a "crystalline" dispersion rather than an amorphous dispersion/solid solution. This hypothesis was confirmed by hot-stage microscopy. It appears that the xylitol/Transcutol™/base formulation has high dissolution rates despite the existence of the drug in a crystalline state. The explanation for this observation is that the crystalline state is likely the salt form rather than the acid form.

The formulation of solid dispersions at dosages up to 40 mg/capsule containing xylitol/Transcutol™/NaOH (or KOH) were shown to have acceptable impurity levels (typically <0.5%) and rapid dissolution (>90% in 10 minutes) in water at 37°C (pH=5.5). Analysis by differential scanning calorimetry and x-ray powder diffraction indicated the existence of crystalline xylitol and crystalline drug. The crystalline nature of the drug was confirmed by observation of the formulation with polarized light microscopy at the melting temperature of xylitol. FTIR analysis of the solid produced when adding the solid dispersion formulation to 0.1N HCl indicated a desirable amorphous acid phase. This suggests that the solid dispersion could have similar bioavailability when compared to the liquid. Advantages of the dispersion formulation include improved manufacturability and ease of fill into hard gel capsules.

### Naproxyn

Another preferred poorly soluble drug substance is Naproxyn. The formulation of Naproxyn was prepared by dissolving 4.6g of Naproxyn in 4.6 g of Transcutol™ (diethylene glycol monoethyl ether) with the addition of 2g of 45% w/v aqueous potassium hydroxide. The Naproxyn dissolved within a few minutes of adding the basic solution. The Naproxyn solution was then added to molten xylitol at 100°C at a ratio of approximately 1:3 by weight. The product was mixed for several minutes, then poured into a crystallizing dish at room temperature. After 24 hours, the product was removed and ground in a mortar and pestle. The final Naproxyn concentration was approximately 10% by weight. The product was a white crystalline powder with a melting point of approximately 90°C.

The dissolution rate using USP Apparatus 2 of the Naproxyn solid dispersion formulation (filled into size 0 hard gelatin capsules) was compared with pure Naproxyn and the potassium salt of Naproxyn under the following conditions:
Dissolution media - 0.01 M phosphate buffer, pH=6
Volume - 1000 ml
RPM - 50
Dosage - 50 mg active for formulation, Naproxyn and Naproxyn potassium salt
Temperature - 37°C.

The dissolution results (3 capsules/formulation) are summarized below:

| **Formulation** | **% Dissolved at 30 minutes** |
|---|---|
| Xylitol Dispersion of Naproxyn Potassium | 97.4 +/- 0.8 |
| Naproxyn | 57.1 +/- 18.5 |
| Naproxyn Potassium | 98.3 +/ 1.0 |

The formulation in the capsules containing the xylitol dispersions dissolved faster than the capsules containing Naproxyn drug substance. The formulation in the capsules containing the potassium salt of Naproxyn dissolved in a similar manner to the xylitol based formulation.

The advantage of the xylitol/Transcutol™/base dispersion versus a formulation containing the acid form of a drug is to cause partial or complete conversion to the salt which will, in general, enhance dissolution rate and solubility. The advantage of the xylitol dispersion versus a formulation containing the salt form is to avoid the potential problems involved with storage of salts (e.g., hygroscopicity). There are several advantages of using xylitol specifically as a carrier. It crystallizes well despite the inclusion of 20% or more drug and Transcutol™. The melting point (95°C) is higher than polyethylene glycols (50-60°C) and there appears to be little melting point depression for xylitol dispersions. In addition, it is highly water soluble, resulting in rapid dissolution and drug release.

**IB)** The Spray Granulation Process
Materials used
SR48692
Xylitol
Lactose Monohydrate
Microcrystalline Cellulose
Dicalcium Phosphate
Croscarmellose Sodium
Sodium Stearyl Fumarate
Magnesium Stearate
Silicon Dioxide
Propylene Glycol
NaOH

### Preparation of Feed Spray

A drug solution was prepared by adding SR48692 to a solvent system containing propylene glycol and aqueous sodium hydroxide in a solvent/drug ratio of about 1.2:1 at a temperature of about 40°C. The sodium hydroxide level was in slight excess relative to drug, i.e. about 1.1:1 molar ratio. The system was mixed for approximately 30 to 60 minutes to insure proper dissolution of the drug. After dissolution was complete, the SR48692 solution was added to a 50% w/w aqueous xylitol solution and mixed for about 10 minutes at about 25°C. The final SR48692 concentration in the spray solution was approximately 8% w/w.

### Fluid Bed Processing

A fluid bed processor was utilized to produce all products. Seed powder was added to the unit and preheated to 40-45°C over a 15 minute period. Seed powders included xylitol, dicalcium phosphate, and lactose monohydrate combined with microcrystalline cellulose. Approximately 700g of seed powder was used. The drug solution was sprayed onto the seed powder at the following conditions:

| Aeromatic Fluid Bed Parameters | |
|---|---|
| **Parameters** | **Setting** |
| Spray Rate | 4cc/min |
| Inlet Air Temp | 60°C |
| Product Temp | 40-45°C |
| Atomization Air Pressure | 1 Bar |
| Air Flow | 40 CMH |

Example 5 illustrates the invention.

### Example 5

| **Feed Spray** | | **Seed Powders** |
|---|---|---|
| SR48692 | 20 g | Xylitol |
| Propylene Glycol | 24 g | Lactose Monohydrate |
| NaOH (35% w/v aq) | 6 g | Dicalcium Phosphate |
| Xylitol | 100 g | Microcrystalline Cellulose |
| Purified Water | 100 g | |

### Addition of Excipients for Tablet/Capsule Formulation

The product containing xylitol as the seed powder was preground with a glass mortar and pestle and screened through a 200 mesh screen prior to adding additional excipients. This product was modified by the addition of 9% microcrystalline cellulose, 0.5% silicon dioxide as glidant, and a lubricant such as 2% w/w sodium stearyl fumarate or 2% w/w magnesium stearate. The product which was prepared by using lactose monohydrate/microcrystalline cellulose in the ratio of 6:1 as the seed powder was modified by dry blending 2% croscarmellose sodium as disintegrant, and 1% sodium stearyl fumarate as the lubricant. The product which was prepared with dicalcium phosphate dihydrate was modified by dry blending 10% corn starch and 4% w/w croscarmellose as a disintegrant.

The product was pressed into tablets or filled into capsules.

### Testing for Impurity

HPLC results were obtained for measurement of impurity levels. The column used was a C18 column with a mobile phase consisting of 0.01M potassium phosphate monobasic (pH=2.5)/acetonitrile (270/730) at a flowrate of 1.0 ml/min. Injection volume was 10 microliters and the detection method was by UV (at 230 nm). For the assay determination, the samples were first diluted in methanol/ammonium hydroxide (99.8/0.2), then further diluted in methanol to obtain a final SR48692 concentration of 1.0 mg/ml. The known impurities were quantitated versus external standards at a concentration of 0.0005 mg/ml. Any unknown impurities were quantified versus an external standard. All impurities greater than 0.01% were added to obtain the total impurity result.

| **Seed Powder** | **Solvent** | **% Impurity** |
|---|---|---|
| Xylitol* | Propylene Glycol | 0.6% |
| Xylitol* | Propylene Glycol | 0.6% |
| Dicalcium. Phosphate Dihydrate* | Propylene Glycol | 0.7% |
| Lactose MH/Microcryst. Cellulose* | Propylene Glycol | 0.5% |
| Lactose MH/Microcryst. Cellulose** | Propylene Glycol | 0.3% |
| Lactose MH/Microcryst. Cellulose** | Propylene Glycol | 0.3% |

| | | |
|---|---|---|
| * Initial drug impurity level = 0.6% | | |
| ** Initial drug impurity level = 0.3% | | |

### Dissolution Screening

The dissolution rate of 10 mg tablets and capsules was measured in pH=7 phosphate buffer (0.005 molar) at 37°C. A Distek USP dissolution system was used with a paddle speed of 75 RPM. Dissolution rate was monitored by UV (Hewlett Packard 8452A) at 260 nm with background correction at 480 nm. The results reported are the average of 2-3 tablets for each formulation.

| **Effect of Seed Powder on Dissolution Rate 10 mg Tablets, 75 RPM, pH=7 buffer (.0005M phosphate)** | | | |
|---|---|---|---|
| | **% Dissolved** | **% Dissolved** | **% Dissolved** |
| **Time (min)** | **Lactose/Microcrystalline Cellulose** | **Xylitol** | **Dicalcium Phosphate** |
| 7.5 | 85% | 69% | 21% |
| 15 | 100% | 95% | 28% |
| 22.5 | 100% | 96% | 39% |
| 30 | 100% | 97% | 55% |

### Concentrated Solutions IIA and IIB

**IIA**) In accordance with this invention, there is provided a pharmaceutical formulation comprising 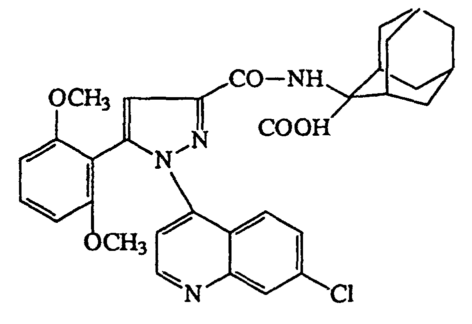 as the active ingredient, polyethylene glycol, NaOH, and H₂O, wherein the mole equivalents of NaOH per mole equivalent of agent is at least about 1.1.

The polyethylene glycol used herein has an average molecular weight of between about 200-100,000 daltons (hereinafter, all molecular weights are expressed in daltons). Moreover, the molecular weight of polyethylene glycol selected affects the type of solution produced. Polyethylene glycol having an average molecular weight from about 200-800, preferably from about 300-700, and most preferably about 400, produces a soft gelatin capsule fill solution that is a liquid. Polyethylene glycol having an average molecular weight from about 800-10,000, preferably from about 2,000-8,000, produces a soft gelatin capsule fill solution that is semisolid, and polyethylene glycol having an average molecular weight between about 10,000-100,000, preferably about 15,000-60,000, produces a soft gelatin capsule fill solution that is solid.

Contemplated equivalents of polyethylene glycol include analogs, such as the polyethylene glycol ethers of various alcohols including but not limited to tetraglycol - the polyethylene glycol ether of tetrahydrofurfuryl alcohol, and copolymers of polyethylene glycol.

The polyethylene glycol can be present in amounts of 60-99%, preferably 70-98% and more preferably 80-95% by weight based on the total weight of the formulation.

The formulation can comprise 0.1-25%, preferably 0.5-20% and more preferably 1-15% by weight water.

The SR48692 can be present in an amount up to 10%, preferably 0.1-9% and more preferably 0.5-7.5% by weight.

The mole equivalents of NaOH present per mole of SR 48692 is at least about 1.1, preferably at least 1.2 and more preferably at least 1.3. Inadequate solubility of the agent was found at mole equivalents of 1.0 and less.

The formulations of this invention can be prepared by adding aqueous NaOH to the polyethylene glycol. The active ingredient SR48692 is added to the PEG-NaOH until a solution is formed. Alternatively, the active ingredient can be dispersed in the PEG with mixing. Thereafter, the NaOH solution can be added resulting in dissolution of the active ingredient. Thereafter, the solution formulations can be encapsulated in a soft gelatin capsule according to techniques known in the art in order to form a pharmaceutical dosage form.

The following examples further illustrate the invention.

### Example 6

A 2.02 kg quantity of a 0.5% w/v sodium hydroxide solution was added to 20.4 kg of polyethylene glycol 400; this mixture was stirred until a clear solution was formed. To the polyethylene glycol 400/sodium hydroxide solution was added 100 g of SR48692; this mixture was stirred until the drug was completely dissolved by visual inspection. The resulting SR48692 solution was shipped to R. P. Scherer for encapsulation into soft gelatin capsules.

### Example 7

A 1.98 kg quantity of a 2.5% sodium hydroxide solution was added to 20.0 kg of polyethylene glycol 400; this mixture was stirred until a clear solution was formed. To the polyethylene glycol 400/sodium hydroxide solution was added 600 g of SR48692; this mixture was stirred until the drug was completely dissolved by visual inspection. The resulting SR48692 solution was shipped to R. P. Scherer for encapsulation into soft gelatin capsules.

### Example 8

A 1.92 kg quantity of a 6.0% w/v sodium hydroxide solution was added to 19.4 kg of polyethylene glycol 400; this mixture was stirred until a clear solution was formed. To the polyethylene glycol 400/sodium hydroxide solution was added 1.50 kg of SR48692; this mixture was stirred until the drug was completely dissolved by visual inspection. The resulting SR48692 solution was shipped to R. P. Scherer for encapsulation into soft gelatin capsules.

| **S R48692 CAPSULE SOFT GELATIN 5MG** | |
|---|---|
| **Ingredient** | **Composition mg/capsule** |
| Polyethylene Glycol 400 | 1020 |
| Sodium Hydroxide | 0.500 |
| Purified Water | 100 |
| SR48692 | 5 |
| Soft Gelatin Capsule, White Opaque | |
| R.P. Scherer Capsule | |
| 18 Oblong (Die Size W18BD) | |
| Gel Formula 005LSMH | |
| Color 911 P | 1 Capsule |

| **SR48692 CAPSULE SOFT GELATIN 30MG** | |
|---|---|
| **Ingredient** | **Composition mg/capsule** |
| Polyethylene Glycol 400 | 1000 |
| Sodium Hydroxide | 2.50 |
| Purified Water | 96.4 |
| SR48692 | 30 |
| Soft Gelatin Capsule, White Opaque | |
| R.P. Scherer Capsule | |
| 18 Oblong (Die Size W18BD) | |
| Gel Formula 005LSMH | |
| Color 911 P | 1 Capsule |

| **SR48692 CAPSULE SOFT GELATIN 75MG** | |
|---|---|
| **Ingredient** | **Composition mg/capsule** |
| Polyethylene Glycol 400 | 969 |
| Sodium Hydroxide | 5.77 |
| Purified Water | 90.4 |
| SR48692 | 75 |
| Soft Gelatin Capsule, White Opaque | |
| R.P. Scherer Capsule | |
| 18 Oblong (Die Size W18BD) | |
| Gel Formula 005LSMH | |
| Color 911 P | 1 Capsule |

**IIB**) In accordance with this embodiment of the present invention, the pharmaceutical formulations comprises a poorly soluble organic acid drug selected from the group consisting of: analgesics, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytic sedatives, astringents, beta-adrenoceptor blocking agents, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, parasympathomimetics, parathyroid calcitonin, prostaglandins, radio-pharmaceuticals, sex hormones, anti-allergic agents, stimulants, anoretics, sympathomimetics, thyroid agents, vasodilators and xanthines; propylene glycol; NaOH and water, wherein the mole equivalent of NaOH per mole equivalent of the poorly soluble drug is at least about 1.1.

Preferred drugs are SR48692 and Naproxyn.

The so-produced drugs are filled into soft gelatin capsules.

### Example 9

| **SR48692 CAPSULE SOFT GELATIN 5MG** | |
|---|---|
| **Ingredient** | **Composition mg/capsule** |
| Propylene Glycol | 1020 |
| Sodium Hydroxide | 0.500 |
| Purified Water | 100 |
| SR48692 | 5 |
| Soft Gelatin Capsule, White Opaque | |
| R.P. Scherer Capsule | |
| 18 Oblong (Die Size W18BD) | |
| Gel Formula 005LSMH | |
| Color 911 P | 1 Capsule |

### Example 10

| **SR48692 CAPSULE SOFT GELATIN 30MG** | |
|---|---|
| **Ingredient** | **Composition mg/capsule** |
| Propylene Glycol | 1000 |
| Sodium Hydroxide | 2.50 |
| Purified Water | 96.4 |
| SR48692 | 30 |
| Soft Gelatin Capsule, White Opaque | |
| R.P. Scherer Capsule | |
| 18 Oblong (Die Size W18BD) | |
| Gel Formula 005LSMH | |
| Color 911 P | 1 Capsule |

### Example 11

| **SR48692 CAPSULE SOFT GELATIN 75MG** | |
|---|---|
| **Ingredient** | **Composition mg/capsule** |
| Propylene Glycol | 969 |
| Sodium Hydroxide | 5.77 |
| Purified Water | 90.4 |
| SR48692 | 75 |
| Soft Gelatin Capsule, White Opaque | |
| R.P. Scherer Capsule | |
| 18 Oblong (Die Size W18BD) | |
| Gel Formula 005LSMH | |
| Color 911 P | 1 Capsule |

### Suspensions IIIA and IIIB

**IIIA**) We have discovered a solvent system for SR48692 which produces a highly concentrated suspension of the active ingredient suitable for a hard gelatin capsule filling.

More specifically, there is provided a pharmaceutical formulation comprising:
0.1 to 40% by weight of SR48692;
0.1 to 40% by weight of Transcutol™;
0.1 to 99% by weight starch;
NaOH; and
H₂O.

The SR48692 can be present in an amount up to 40%, preferably 0.1 - 35% and more preferably 0.5 - 30% by weight.

The Transcutol is present in an amount of 0.1 to 40%, preferably 0.5 to 35% and most preferably, 1 to 30% by weight of the solid dispersion. Transcutol™ is commercially available and/or can be prepared by techniques well known to those skilled in the art.

The starch can be present in an amount of 0.1 - 99% by weight. Starches which have previously been used in pharmaceutical formulations are preferred for use herein. The starch can be pre-gelatinized, i.e., chemically and/or mechanically processed to rupture all or part of the granules in the presence of water and subsequently dried. Suitable USP/NF starches are described in the Handbook of Pharmaceutical Excipients, Second Edition published jointly by the American Pharmaceutical Association and the Pharmaceutical Society of Great Britain, The Pharmaceutical Press, 1994. A preferred starch is Starch 1500, commercially available from Colorcon.

The suspension can comprise 0.1 - 20%, preferably 0.5 - 15% and more preferably 1 - 13% water. Higher amounts of water tend to soften and/or dissolve the gelatin capsules shells.

The NaOH is present in an amount such that the mole equivalents of NaOH per mole equivalent of SR48692 is from about 0.5 to 1.5.

The polyethylene glycol can be present in amounts of 0 - 90%, preferably 1 - 85% and more preferably 2 - 80% by weight based on the total weight of the formulation.

The polyethylene glycol optionally used herein has an average molecular weight of between about 200-100,000 daltons (hereinafter, all molecular weights are expressed in daltons). Moreover, the molecular weight of polyethylene glycol selected affects the type of solution produced. Polyethylene glycol having an average molecular weight from about 200-800, preferably from about 300-700, and most preferably about 400, produces a hard gelatin capsule fill solution that is a liquid. These low molecular weights are preferred because the PEG is miscible with the Transcutol™. High molecular weights may disadvantageously require heat to melt and/or dissolve in the Transcutol™. Polyethylene glycol having an average molecular weight from about 800-10,000, preferably from about 2,000-8,000, produces a hard gelatin capsule fill solution that is semisolid, and polyethylene glycol having an average molecular weight between about 10,000-100,000, preferably about 15,000-60,000, produces a hard gelatin capsule fill solution that is solid.

Contemplated equivalents of polyethylene glycol include analogs, such as the polyethylene glycol ethers of various alcohols including but not limited to tetraglycol - the polyethylene glycol ether of tetrahydrofurfuryl alcohol, and copolymers of polyethylene glycol.

The suspensions of this invention can be prepared by adding the SR48692 to the solvent Transcutol, optionally in combination with PEG. The SR48692 is then dissolved by adding aqueous NaOH. The suspension is obtained by adding water which causes a controlled precipitation. Addition of starch to the suspension permits the formulation to be filled into hard gelatin capsules preserving the integrity of the capsule shells.

The following example further illustrates the invention.

### Example 12

12g of PEG 400 and 12g of diethyleneglycol monoethyl ether (Transcutol™) were mixed with a magnetic stir plate apparatus at 20°C. Next, 16g of SR48692 were added along with 4.7g of aqueous sodium hydroxide (35% w/v). The system was mixed at 20°C which caused a large proportion of SR48692 to dissolve after 10 minutes. 4g of purified water was then added and the system was mixed for several hours as a precipitate formed (until a concentrated suspension of drug was achieved). The product was mixed with 12g of pre-gelatinized starch with a mortar and pestle to obtain the final formulation. The product was filled into size #0 hard gelatin capsules at 150mg drug/capsule. After 8 months at room temperature the estimated total impurity level of the product was 0.4%, well within acceptable levels.

**IIIB**) In this embodiment of the present invention a pharmaceutical composition in the form of a suspension is provided for filling hard gelatin capsules comprising:
a) 0.1 to 40% by weight of SR48692;
b) 0.1 to 40% by weight propylene glycol;
c) 0.1 o 99% by weight of starch;
d) 0.1 to 20% water; and
e) NaOH,
wherein the mole equivalent of NaOH per mole equivalent of the poorly soluble drug is from 0.5 to 1.5.

The suspension can be prepared by adding the poorly soluble drug substance to propylene glycol and dissolving the same by adding aqueous NaOH. The suspension is obtained by adding water which causes a controlled precipitation. Addition of the starch to the suspension permits the formulation to be filled into hard gelatin capsules preserving the integrity of the capsule shells.

Having described the invention with reference to its preferred embodiments, it is to be understood that modifications within the scope of the invention will be apparent to those skilled in the art.

## Claims

1. A pharmaceutical formulation in the form of a solid dispersion comprising:
a) a poorly soluble organic acid drug substance, having a solubility in an aqueous medium of less than 10mg/ml, selected from the group consisting of:
analgesics, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytic sedatives, astringents, beta-adrenoceptor blocking agents, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, parasympathomimetics, parathyroid calcitonin, prostaglandins, radio-pharmaceuticals, sex hormones, anti-allergic agents, stimulants, anoretics, sympathomimetics, thyroid agents, vasodilators and xanthines;
b) propylene glycol;
c) a diluent selected from the group consisting of xylitol, dicalcium phosphate dihydrate, and lactose monohydrate and/or microcrystalline cellulose.

2. A pharmaceutical formulation in the form of a solid dispersion comprising:
a) a poorly soluble drug substance, having a solubility in an aqueous medium of less than 10mg/ml;
b) xylitol; and
c) diethyleneglycol monoethyl ether.

3. The pharmaceutical formulation of claim 1 or claim 2 further comprising a base.

4. The pharmaceutical formulation of any one of claims 1 to 3 wherein said poorly soluble drug substance is SR48692.

5. The pharmaceutical formulation of any one of claims 1 to 3 wherein said poorly soluble drug substance is Naproxyn.

6. A process of preparing a pharmaceutical formulation in the form of a solid dispersion comprising the steps of:
a) dissolving a poorly soluble organic acid drug substance, having a solubility in an aqueous medium of less than 10mg/ml, in aqueous sodium hydroxide/propylene glycol mixture to form a solution wherein said poorly soluble drug substance is selected from the group consisting of:
analgesics, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytic sedatives, astringents, beta-adrenoceptor blocking agents, cardiac inotropic agents, contrast media, corticosteroids, cough suppressants, diagnostic agents, diagnostic imaging agents, diuretics, dopaminergics, haemostatics, immunological agents, lipid regulating agents, muscle reiaxants, parasympathomimetics, parathyroid calcitonin, prostaglandins, radio-pharmaceuticals, sex hormones, anti-allergic agents, stimulants, anoretics, sympathomimetics, thyroid agents, vasodilators and xanthines;
and
b) adding the solution to a diluent selected from the group consisting of xylitol, dicalcium phosphate dihydrate and lactose monohydrate and/or microcrystalline cellulose.

7. The process of claim 6 further comprising adding water or aqueous xylitol to said sodium hydroxide/propylene glycol mixture in step (a).

8. The process of claim 6 or 7 wherein the poorly soluble drug substance is SR48692.

9. The process of claim 6 or 7 wherein the poorly soluble drug substance is Naproxyn.

10. A concentrated drug solution for soft gelatin capsule filling consisting essentially of:
a) a poorly soluble organic acid drug substance, having a solubility in an aqueous medium of less than 10mg/ml, selected from the group consisting of analgesics, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytic sedatives, astringents, beta-adrenoceptor blocking agents, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, parasympathomimetics, parathyroid calcitonin, prostaglandins, radio-pharmaceuticals, sex hormones, anti-allergic agents, stimulants, anoretics, sympathomimetics, thyroid agents, vasodilators and xanthines;
b) propylene glycol;
c) sodium hydroxide; and
d) water; wherein the mole equivalent of sodium hydroxide per mole equivalent of the poorly soluble drug is at least 1.1.

11. The drug solution of claim 10 wherein the ratio of mole equivalent of sodium hydroxide per mole equivalent of said poorly soluble drug is at least 1.2.

12. The drug solution according to claim 10 or 11 wherein said poorly soluble drug substance is SR48692.

13. The drug solution according to claim 10 or 11 wherein said poorly soluble drug is Naproxyn.

14. A pharmaceutical formulation in a highly concentrated suspension form for a hard gelatin capsule filling comprising:
0.1 to 40% by weight of SR48692 as the active ingredient;
0.1 to 40% by weight propylene glycol;
0.1 to 99% by weight of a diluent selected from the group consisting of starch; microcrystalline cellulose and lactose; and
sodium hydroxide and water, wherein the mole equivalent of sodium hydroxide per mole equivalent of the poorly soluble drug substance is from 0.5 to 1.5.

15. A pharmaceutical formulation comprising 0.1 to 40% by weight of SR48692 as the active ingredient; 0.1 to 40% by weight diethylene glycol monoethyl ether; 0.1-99% by weight starch; NaOH and water, wherein the mole equivalent of NaOH per mole equivalent of active ingredient is from 0.5 to 1.5.

16. The pharmaceutical formulation of claim 14 or 15 wherein said active ingredient is present in an amount of 1 to 30% by weight.

17. The pharmaceutical formulation of any of claims 14 to 16 further including polyethylene glycol, a polyethylene glycol ether of an alcohol or a copolymer of polyethylene glycol.

18. The pharmaceutical formulation of any claims 14 to 17 wherein the water is present in an amount from 0.1 to 20% by weight.

19. A method of preparing a pharmaceutical formulation in the form of a solid dispersion comprising the steps of:
a) dissolving a poorly soluble drug substance in diethylene glycol monoethylether; and
b) adding the solution to xylitol.

20. A pharmaceutical formulation comprising from about 0.1 to about 10% w/w of SR48692 as the active ingredient, polyethylene glycol, a polyethylene glycolether of an alcohol or a copolymer of polyethylene glycol, NaOH, and water, wherein the mole equivalents of NaOH per mole equivalent of active ingredient is at least 1.1.

21. The formulation of claim 20 wherein the ratio of mole equivalents of NaOH per mole equivalent of active ingredient is at least 1.2.

22. The formulation of claims 20 or 21 wherein the polyethylene glycol polyethylene glycol ether of an alcohol or copolymer of polyethylene glycol has a molecular weight of from 200-800.

## Patentansprüche

1. Pharmazeutische Formulierung in Form einer festen Dispersion, umfassend:
a) eine schlecht lösliche organische Säure-Arzneistoffsubstanz mit einer Löslichkeit in einem wässrigen Medium von weniger als 10 mg/ml, ausgewählt aus der Gruppe, bestehend aus:
Analgetika, entzündungshemmenden Mitteln, Anthelmintika, antiarrhythmischen Mitteln, Antibiotika, Gerinnungshemmern, Antidepressiva, Antidiabetes-Mitteln, Antiepileptika, Antihistaminen, blutdrucksenkenden Mitteln, antimuskarinen Mitteln, antimykobakteriellen Mitteln, antineoplastischen Mitteln, Immunosuppressantien, Antithyroidmitteln, antiviralen Mitteln, angstlösenden Sedativa, Adstringenzien, Betaadrenozeptor blockierenden Mitteln, inotropen Herzmitteln, Kontrastmedien, Corticosteroiden, hustenlindernden Mitteln, diagnostischen Mitteln, diagnostischen Abbildungsmitteln, Diuretika, Dopaminergika, Hämostatika, immunologischen Mitteln, lipidregulierenden Mitteln, Muskelrelaxanzien, Parasympathomimetika, Parathyroidcalcitonin, Prosta-glandinen, Radiopharmazeutika, Sexualhormonen, antiallergischen Mitteln, Stimulanzien, Anoretika, Sympathomimetika, Thyroidznitteln, Vasodilatatoren und Xanthinen;
b) Propylenglykol;
c) ein Verdünnungsmittel, ausgewählt aus der Gruppe, bestehend aus Xylit, Dicalciumphosphatdihydrat und Laktosemonohydrat und/oder mikrokristalliner Zellulose.

2. Pharmazeutische Formulierung in Form einer festen Dispersion, umfassend:
a) eine schlecht lösliche Arzneistoffsubstanz mit einer Löslichkeit in einem wässrigen Medium von weniger als 10 mg/ml,
b) Xylit und
c) Diethylenglykolmonoethylether.

3. Pharmazeutische Formulierung nach Anspruch 1 oder Anspruch 2, weiterhin umfassend eine Base.

4. Pharmazeutische Formulierung nach einem der Ansprüche 1 bis 3, worin die schlecht lösliche Arzneistoffsubstanz SR48692 ist.

5. Pharmazeutische Formulierung nach einem der Ansprüche 1 bis 3, worin die schlecht lösliche Arzneistoffsubstanz Naproxyn ist.

6. Verfahren zum Herstellen einer pharmazeutischen Formulierung in Form einer festen Dispersion, umfassend die Schritte von:
a) Auflösen einer schlecht löslichen organischen Säure-Arzneistoffsubstanz mit einer Löslichkeit in einem wässrigen Medium von weniger als 10 mg/ml in wässrigem Natriumhydroxid/Propylenglykol-Gemisch unter Bildung einer Lösung, wobei die schlecht lösliche Arzneistoffsubstanz ausgewählt ist aus der Gruppe, bestehend aus:
Analgetika, entzündungshemmenden Mitteln, Anthelmintika, antiarrhythmischen Mitteln, Antibiotika, Gerinnungshemmern, Antidepressiva, Antidiabetes-Mitteln, Antiepileptika, Antihistaminen, blutdrucksenkenden Mitteln, antimuskarinen Mitteln, antimykobakteriellen Mitteln, antineoplastischen Mitteln, Immunosuppressantien, Antithyroidmitteln, antiviralen Mitteln, angstlösenden Sedativa, Adstringenzien, Betaadrenozeptor blockierenden Mitteln, inotropen Herzmitteln, Kontrastmedien, Corticosteroiden, hustenlindernden Mitteln, diagnostischen Mitteln, diagnostischen Abbildungsmitteln, Diuretika, Dopaminergika, Hämostatika, immunologischen Mitteln, lipidregulierenden Mitteln, Muskelrelaxanzien, Parasympathomimetika, Parathyroidcalcitonin, Prosta-glandinen, Radiopharmazeutika, Sexualhormonen, antiallergischen Mitteln, Stimulanzien, Anoretika, Sympathomimetika, Thyroidmitteln, Vasodilatatoren und Xanthinen; und
b) Zugabe der Lösung zu einem Verdünnungsmittel, ausgewählt aus der Gruppe, bestehend aus Xylit, Dicalciumphosphatdihydrat und Laktosemonohydrat und/oder mikrokristalliner Zellulose.

7. Verfahren nach Anspruch 6, weiterhin umfassend Zusetzen von Wasser oder wässrigem Xylit zu dem Natriumhydroxid/Propylenglykol-Gemisch in Schritt (a).

8. Verfahren nach Anspruch 6 oder 7, wobei die schlecht lösliche Arzneistoffsubstanz SR48692 ist.

9. Verfahren nach Anspruch 6 oder 7, wobei die schlecht lösliche Arzneistoffsubstanz Naproxyn ist.

10. Konzentrierte Arzneistofflösung für eine Weichgelatinekapselfüllung, bestehend im Wesentlichen aus:
a) einer schlecht löslichen organischen Säure-Arzneistoffsubstanz mit einer Löslichkeit in einem wässrigen Medium von weniger als 10 mg/ml, ausgewählt aus der Gruppe, bestehend aus Analgetika, entzündungshemmenden Mitteln, Anthelmintika, antiarrhythmischen Mitteln, Antibiotika, Gerinnungshemmern, Antidepressiva, Antidiabetes-Mitteln, Anti-epileptika, Antihistaminen, blutdrucksenkenden Mitteln, antimuskarinen Mitteln, antimykobakteriellen Mitteln, antineoplastischen Mitteln, Immunosuppressantien, Antithyroidmitteln, antiviralen Mitteln, angstlösenden Sedativa, Adstringenzien, Betaadrenozeptor blockierenden Mitteln, inotropen Herzmitteln, Kontrastmedien, Corticosteroiden, hustenlindernden Mitteln, diagnostischen Mitteln, diagnostischen Abbildungsmitteln, Diuretika, Dopaminergika, Hämostatika, immunologischen Mitteln, lipidregulierenden Mitteln, Muskelrelaxanzien, Parasympathomimetika, Parathyroidcalcitonin, Prostaglandinen, Radiopharmazeutika, Sexualhormonen, antiallergischen Mitteln, Stimulanzien, Anoretika, Sympathomimetika, Thyroidmitteln, Vasodilatatoren und Xanthinen;
b) Propylenglykol;
c) Natriumhydroxid und
d) Wasser, worin das Moläquivalent von Natriumhydroxid pro Moläquivalent des schlecht löslichen Arzneistoffs mindestens 1,1 ist.

11. Arzneistofflösung nach Anspruch 10, worin das Moläquivalent-Verhältnis von Natriumhydroxid pro Moläquivalent des schlecht löslichen Arzneistoffs mindestens 1,2 ist.

12. Arzneistofflösung nach Anspruch 10 oder 11, worin die schlecht lösliche Arzneistoffsubstanz SR48692 ist.

13. Arzneistofflösung nach Anspruch 10 oder 11, worin der schlecht lösliche Arzneistoff Naproxyn ist.

14. Pharmazeutische Formulierung in hochkonzentrierter Suspensionsform für eine Hartgelatinekapselfüllung, umfassend
0,1 bis 40 Gew.-% SR48692 als den Wirkstoff;
0,1 bis 40 Gew.-% Propylenglykol;
0,1 bis 99 Gew.-% eines Verdünnungsmittels, ausgewählt aus der Gruppe, bestehend aus Stärke, mikrokristalliner Zellulose und Laktose und
Natriumhydroxid und Wasser, worin das Moläquivalent von Natriumhydroxid pro Moläquivalent der schlecht löslichen Arzneistoffsubstanz 0,5 bis 1,5 ist.

15. Pharmazeutische Formulierung, umfassend 0,1 bis 40 Gew.-% SR48692 als Wirkstoff; 0,1 bis 40 Gew.-% Diethylenglykolmonoethylether; 0,1 bis 99 Gew.-% Stärke; NaOH und Wasser, worin das Moläquivalent NaOH pro Moläquivalent Wirkstoff 0,5 bis 1,5 ist.

16. Pharmazeutische Formulierung nach Anspruch 14 oder 15, worin der Wirkstoff in einer Menge von 1 bis 30 Gew.-% vorliegt.

17. Pharmazeutische Formulierung nach einem der Ansprüche 14 bis 16, die weiterhin Polyethylenglykol, einen Polyethylenglykolether eines Alkohols oder ein Copolymer von Polyethylenglykol einschließt.

18. Pharmazeutische Formulierung nach einem der Ansprüche 14 bis 17, worin das Wasser in einer Menge von 0,1 bis 20 Gew.-% vorliegt.

19. Verfahren zum Herstellen einer pharmazeutischen Formulierung in Form einer festen Dispersion, umfassend die Schritte von:
a) Auflösen einer schlecht löslichen Arzneistoffsubstanz in Diethylenglykolmonoethylether und
b) Zusetzen der Lösung zu Xylit.

20. Pharmazeutische Formulierung, umfassend etwa 0,1 bis etwa 10 % Gewicht/Gewicht SR48692 als Wirkstoff, Polyethylenglykol, einen Polyethylenglykolether eines Alkohols oder ein Copolymer von Polyethylenglykol, NaOH und Wasser, worin die Moläquivalente von NaOH pro Moläquivalent Wirkstoff mindestens 1,1 ist.

21. Formulierung nach Anspruch 20, worin das Verhältnis von Moläquivalenten NaOH pro Moläquivalent Wirkstoff mindestens 1,2 ist.

22. Formulierung nach Ansprüchen 20 oder 21, worin das Polyethylenglykol, der Polyethylenglykolether eines Alkohols oder das Copolymer von Polyethylenglykol ein Molekulargewicht von 200-800 aufweist.

## Revendications

1. Formulation pharmaceutique sous forme d'une dispersion solide, comprenant :
a) une substance médicamenteuse d'acide organique peu soluble, ayant une solubilité dans un milieu aqueux inférieure à 10 mg/ml, choisie parmi le groupe constitué par :
les analgésiques, les agents anti-inflammatoires, les anthelminthiques, les agents antiarythmiques, les antibiotiques, les anticoagulants, les antidépresseurs, les agents antidiabétiques, les anti-convulsivants, les antihistaminiques, les agents antihypertenseurs, les agents antimuscariniques, les agents anti-mycobactériens, les agents antinéoplasiques, les immunosuppresseurs, les agents antithyroïdiens, les agents anti-viraux, les sédatifs anxiolytiques, les astringents, les agents bloquant l'adrénocepteur bêta, les agents inotropes cardiaques, les produits de contraste, les corticostéroïdes, les antitussifs, les agents de diagnostic, les agents d'imagerie diagnostique, les diurétiques, les agents dopaminergiques, les hémostatiques, les agents immunologiques, les agents de régulation des lipides, les myorelaxants, les parasympathomimétiques, la calcitonine parathyroïdienne, les prostaglandines, les produits radiopharmaceutiques, les hormones sexuelles, les agents anti-allergiques, les stimulants, les anoréxigènes, les sympathomimétiques, les agents thyroïdiens, les vasodilatateurs et les xanthines ;
b) du propylène glycol ;
c) un diluant choisi parmi le groupe constitué par le xylitol, le phosphate dicalcique dihydraté, et le lactose monohydraté et/ou la cellulose microcristalline.

2. Formulation pharmaceutique sous forme d'une dispersion solide, comprenant :
a) une substance médicamenteuse peu soluble, ayant une solubilité dans un milieu aqueux inférieure à 10 mg/ml ;
b) du xylitol ; et
c) de l'éther monoéthylique de diéthylène glycol.

3. Formulation pharmaceutique selon la revendication 1 ou la revendication 2, comprenant en outre une base.

4. Formulation pharmaceutique selon l'une quelconque des revendications 1 à 3, dans laquelle ladite substance médicamenteuse peu soluble est le SR48692.

5. Formulation pharmaceutique selon l'une quelconque des revendications 1 à 3, dans laquelle ladite substance médicamenteuse peu soluble est le Naproxyn.

6. Procédé de préparation d'une formulation pharmaceutique sous forme d'une dispersion solide, comprenant les étapes :
a) de solubilisation d'une substance médicamenteuse d'acide organique peu soluble, ayant une solubilité dans un milieu aqueux inférieure à 10 mg/ml, dans un mélange d'hydroxyde de sodium aqueux/propylène glycol pour former une solution dans laquelle ladite substance médicamenteuse peu soluble est choisie parmi le groupe constitué par :
les analgésiques, les agents anti-inflammatoires, les anthelminthiques, les agents antiarythmiques, les antibiotiques, les anticoagulants, les antidépresseurs, les agents antidiabétiques, les anti-convulsivants, les antihistaminiques, les agents antihypertenseurs, les agents antimuscariniques, les agents anti-mycobactériens, les agents antinéoplasiques, les immunosuppresseurs, les agents antithyroïdiens, les agents anti-viraux, les sédatifs anxiolytiques, les astringents, les agents bloquant l'adrénocepteur bêta, les agents inotropes cardiaques, les produits de contraste, les corticostéroïdes, les antitussifs, les agents de diagnostic, les agents d'imagerie diagnostique, les diurétiques, les agents dopaminergiques, les hémostatiques, les agents immunologiques, les agents de régulation des lipides, les myorelaxants, les parasympathomimétiques, la calcitonine parathyroïdienne, les prostaglandines, les produits radiopharmaceutiques, les hormones sexuelles, les agents anti-allergiques, les stimulants, les anoréxigènes, les sympathomimétiques, les agents thyroïdiens, les vasodilatateurs et les xanthines ;
et
b) d'addition de la solution à un diluant choisi parmi le groupe constitué par le xylitol, le phosphate dicalcique dihydraté, et le lactose monohydraté et/ou la cellulose microcristalline.

7. Procédé selon la revendication 6, comprenant en outre l'addition d'eau ou de xylitol aqueux audit mélange d'hydroxyde de sodium/propylène glycol dans l'étape (a).

8. Procédé selon la revendication 6 ou 7, dans lequel la substance médicamenteuse peu soluble est le SR48692.

9. Procédé selon la revendication 6 ou 7, dans lequel la substance médicamenteuse peu soluble est le Naproxyn.

10. Solution médicamenteuse concentrée destinée au remplissage d'une capsule en gélatine molle, constituée essentiellement
a) d'une substance médicamenteuse d'acide organique peu soluble, ayant une solubilité dans un milieu aqueux inférieure à 10 mg/ml, choisie parmi le groupe constitué par les analgésiques, les agents anti-inflammatoires, les anthelminthiques, les agents antiarythmiques, les antibiotiques, les anticoagulants, les antidépresseurs, les agents antidiabétiques, les anti-convulsivants, les antihistaminiques, les agents antihypertenseurs, les agents antimuscariniques, les agents anti-mycobactériens, les agents antinéoplasiques, les immunosuppresseurs, les agents antithyroïdiens, les agents anti-viraux, les sédatifs anxiolytiques, les astringents, les agents bloquant l'adrénocepteur bêta, les agents inotropes cardiaques, les produits de contraste, les corticostéroïdes, les antitussifs, les agents de diagnostic, les agents d'imagerie diagnostique, les diurétiques, les agents dopaminergiques, les hémostatiques, les agents immunologiques, les agents de régulation des lipides, les myorelaxants, les parasympathomimétiques, la calcitonine parathyroïdienne, les prostaglandines, les produits radiopharmaceutiques, les hormones sexuelles, les agents anti-allergiques, les stimulants, les anorexigènes, les sympathomimétiques, les agents thyroïdiens, les vasodilatateurs et les xanthines ;
b) de propylène glycol ;
c) d'hydroxyde de sodium ; et
d) d'eau ; dans laquelle l'équivalent molaire d'hydroxyde de sodium par équivalent molaire du médicament peu soluble est d'au moins 1,1.

11. Solution médicamenteuse selon la revendication 10, dans laquelle le rapport de l'équivalent molaire d'hydroxyde de sodium à l'équivalent molaire dudit médicament peu soluble est d'au moins 1,2.

12. Solution médicamenteuse selon la revendication 10 ou 11, dans laquelle ladite substance médicamenteuse peu soluble est le SR48692.

13. Solution médicamenteuse selon la revendication 10 ou 11, dans laquelle ledit médicament peu soluble est le Naproxyn.

14. Formulation pharmaceutique sous forme de suspension hautement concentrée destinée au remplissage d'une capsule de gélatine dure, comprenant :
de 0,1 à 40% en poids de SR48692 comme ingrédient actif ;
de 0,1 à 40% en poids de propylène glycol ;
de 0,1 à 99% en poids d'un diluant choisi parmi le groupe constitué par l'amidon, la cellulose microcristalline et le lactose ; et
de l'hydroxyde de sodium et de l'eau, dans laquelle l'équivalent molaire d'hydroxyde de sodium par équivalent molaire de substance médicamenteuse peu soluble va de 0,5 à 1,5.

15. Formulation pharmaceutique comprenant de 0,1 à 40% en poids de SR48692 comme ingrédient actif ; de 0,1 à 40% en poids d'éther monoéthylique de diéthylène glycol ; 0,1-99% en poids d'amidon ; du NaOH et de l'eau, dans laquelle l'équivalent molaire de NaOH par équivalent molaire d'ingrédient actif va de 0,5 à 1,5.

16. Formulation pharmaceutique selon la revendication 14 ou 15, dans laquelle ledit ingrédient actif est présent en une quantité de 1 à 30% en poids.

17. Formulation pharmaceutique selon l'une quelconque des revendications 14 à 16, comportant en outre du polyéthylène glycol, un éther de polyéthylène glycol et d'un alcool ou un copolymère de polyéthylène glycol.

18. Formulation pharmaceutique selon l'une quelconque des revendications 14 à 17, dans laquelle l'eau est présente en une quantité de 0,1 à 20% en poids.

19. Méthode de préparation d'une formulation pharmaceutique sous forme d'une dispersion solide, comprenant les étapes :
a) de solubilisation d'une substance médicamenteuse peu soluble dans l'éther monoéthylique de diéthylène glycol ; et
b) d'addition de la solution à du xylitol.

20. Formulation pharmaceutique comprenant d'environ 0,1 à environ 10% p/p de SR48692 comme ingrédient actif, du polyéthylène glycol, un éther de polyéthylène glycol et d'un alcool ou un copolymère de polyéthylène glycol, du NaOH et de l'eau, dans laquelle l'équivalent molaire de NaOH par équivalent molaire d'ingrédient actif est d'au moins 1,1.

21. Formulation selon la revendication 20, dans laquelle le rapport de l'équivalent molaire de NaOH à l'équivalent molaire d'ingrédient actif est d'au moins 1,2.

22. Formulation selon la revendication 20 ou 21, dans laquelle le polyéthylène glycol, l'éther de polyéthylène glycol et d'un alcool ou le copolymère de polyéthylène glycol a un poids moléculaire de 200-800.
